(19) Europäisches Patentamt
European Patent Office
Office européen des brevets

(11) Veröffentlichungsnummer : **0 378 769 B1**

(12) # EUROPÄISCHE PATENTSCHRIFT

(45) Veröffentlichungstag der Patentschrift : **17.02.93 Patentblatt 93/07**

(51) Int. Cl.$^5$ : **C07C 51/58,** C07C 59/135

(21) Anmeldenummer : **89120271.5**

(22) Anmeldetag : **02.11.89**

(54) **Verfahren zur Dimerisierung von Hexafluorpropenoxid.**

(30) Priorität : **14.01.89 DE 3901002**

(43) Veröffentlichungstag der Anmeldung : **25.07.90 Patentblatt 90/30**

(45) Bekanntmachung des Hinweises auf die Patenterteilung : **17.02.93 Patentblatt 93/07**

(84) Benannte Vertragsstaaten : **BE CH DE ES FR GB IT LI NL**

(56) Entgegenhaltungen :
**DE-A- 2 627 986**
**DE-A- 2 756 919**

(56) Entgegenhaltungen :
**CHEMISTRY LETTERS, Nr. 7, Juli 1980, S. 843-846, The Chemical Society of Japan, Tokio, JP; H. KAWA et al: "Optically ActivePerfluoro-2-Propoxypropionic Acid"**
**PATENT ABSTRACTS OF JAPAN, Band 6, Nr. 139 (C-116)(1017), 28. Juli 1982; & JP-A-57 64641**

(73) Patentinhaber : **HOECHST AKTIENGESELLSCHAFT**
**Postfach 80 03 20**
**W-6230 Frankfurt am Main 80 (DE)**

(72) Erfinder : **Siegemund, Günter Dr.**
**Frankfurter Strasse 21**
**W-6238 Hofheim am Taunus (DE)**
Erfinder : **Finke, Manfred Dr.**
**Behringstrasse 25**
**W-6233 Kelkheim (Taunus) (DE)**

**EP 0 378 769 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung des Dimeren von Hexafluorpropenoxid (HFPO).

Literaturbekannt ist die Oligomerisierung von Hexafluorpropenoxid, die zu Säurefluoriden der Formel I mit breiter Molekulargewichtsverteilung (n= 0 bis 30) führt (Angew. Chemie 97, 164 (1985)

$$C_2F_5(CF_2\text{-}O\text{-}\overset{CF_3}{\overset{|}{C}F})_n\text{-}COF \qquad (I) \qquad n= 0,\ 1,\ 2,\ ...$$

Vorgeschlagen wurden auch schon Katalysatorsysteme, die den Selektivitätsschwerpunkt der Oligomerisierung von Hexafluorpropenoxid auf das Dimere der Formel I mit n= 1 lenken. Hierbei führt Silbernitrat als Katalysator in Acetonitril bis zu einer Ausbeute von 86 % zum HFPO-Dimeren (DE-A 20 26 669); der Katalysator ist aber - wie die meisten Ag-Derivate - lichtempfindlich und entwickelt nitrose Gase.

Der Nachteil des Katalysatorsystems $CuCl/CuCl_2$/Acrylnitril in Acetonitril als Lösemittel, das ebenfalls mit hoher Ausbeute zu dimerem HFPO filhrt, ist die Verwendung des cancerogen verdächtigen Acrylnitrils (DE-A 29 24 385).

Darüberhinaus werden hohe Anforderungen an die Temperaturkonstanz während der Reaktion gestellt. Die Einhaltung einer Reaktionstemperatur von -20°C ist unbedingt erforderlich, wenn Cäsiumfluorid in Gegenwart protonenaktiver Verbindungen als selektiver Dimerisierungskatalysator für HFPO wirken soll, was bei technischen Anwendungen nachteilig ist. Außerdem ist Cäsiumfluorid wegen seiner hygroskopischen Eigenschaften schwer zu handhaben und stellt wegen des partiellen Austrags in das Oligomergemisch ein sehr teures Katalysatorsystem dar. (JP-A 62-195 345)

Weiterhin ist bekannt, daß tertiäre Amine oder N,N-Dialkylaniline selbst unter autogenem Druck eine sehr geringe Katalyse bewirken (DE-C 1 645 115) bzw. als unwirksam beschrieben wurden (EP-A 0 203 466). Erst ihre Kombination mit Tetramethylharnstoff ruft eine mäßige Katalyse zu dimerem HFPO hervor.

Es bestand daher die Aufgabe, ein Katalysatorsystem zu finden, das HFPO mit hoher Selektivität dimerisiert und gleichzeitig die Nachteile der Katalysatorsysteme gemäß dem Stand der Technik nicht aufweist.

Gegenstand der Erfindung ist ein Verfahren zur Herstellung von Säurefluoriden der allgemeinen Formel

$$C_2F_5(CF_2\text{-}O\text{-}\overset{CF_3}{\overset{|}{C}F})_nCOF \qquad (I)$$

in der n im wesentlichen die Zahl 1 darstellt das in einem aprotischen polaren Lösungsmittel in Gegenwart eines Katalysators durchgeführt wird, der aus einem Metallsalz der Elemente der 1. Übergangsmetallreihe des Periodischen Systems und einem tertiären Diamin der allgemeinen Formel

$$R^1R^2N\text{-}R\text{-}NR^3R^4 \qquad (II)$$

besteht, in der R einen unverzweigten oder verzweigten, gesattigten oder ungesattigten aliphatischen Kohlenwasserstoffrest mit 1 bis 12, vorzugsweise 1 bis 4 C-Atomen darstellt, der gegebenenfalls mindestens einen bezüglich der Reaktionskomponenten inerten Heterosubstituenten oder ein Heterokettenglied enthält, $R^1$ bis $R^4$ unabhängig voneinander aliphatische oder cycloaliphatische gesättigte oder ungesättigte Kohlenwasserstoffreste mit 1 bis 12, vorzugsweise 1 bis 6 C-Atomen sind, die gegebenenfalls Jeweils mindestens einen bezüglich der Reaktionskomponenten inerten Heterosubstituenten oder ein Heterokettenglied enthalten, wobei gegebenenfalls jeweils zwei der Reste $R^1$ bis $R^4$ zusätzlich über einen Kohlenwasserstoffrest oder mindestens ein heteroatom verknüpft sind.

Heterosubstituenten, Heteroatome oder Heterokettenglieder sind Gruppierungen, die im allgemeinen Stickstoff oder Sauerstoff als Heteroatome enthalten.

Die Aussage, daß n im wesentlichen die Zahl 1 darstellt, beinhaltet, daß die Molekulargewichtsverteilung eng ist. Natürlich können die erhaltenen Säurefluoride der Formel (I) auch solche Zusammensetzungen aufweisen, bei denen n neben der Zahl 1 auch Zahlen von null bis 30 darstellt; jedoch kommen diese Verbindungen nur im untergeordneten Maße vor und werden als Verunreinigungen angesehen.

Der Vorteil des in dem Verfahren gemäß der Erfindung verwendeten Katalysatorsystems besteht darin, daß dieses die oben beschriebenen, mit dem Stand der Technik verbundenen Nachteile nicht besitzt und bezüglich des Selektivitätsschwerpunktes auf dem HFPO-Dimeren und der Oligomerisierungsaktivität ein dem

Stand der Technik überlegenes System darstellt. Vorteilhaft sind weiterhin die hohe Lebensdauer des Katalysators und die sehr geringe Löslichkeit der Katalysatorspecies in der Produktphase, die es erlaubt, das Katalysatorsystem mehrmals zu benutzen.

Im allgemeinen wird eine Verfahrensweise ausgewählt, bei der zunächst das Metallsalz in einem aprotisch polaren Lösemittel gelöst und anschließend das Diamin zugetropft oder portionsweise zugegeben wird, wobei im allgemeinen ein Farbwechsel die Bildung des Katalysatorsystems anzeigt. Im Anschluß daran wird unter Rühren und Kühlen HFPO mit einer solchen Geschwindigkeit eingegast, daß ein gewünschter Betriebsdruck nicht überschritten wird. Nach Abschluß der Reaktion wird die Produktphase abgetrennt und analysiert.

Als Metallsalze werden Fluoride, Chloride, Bromide und Jodide sowie Cyanide, Rhodanide und Acetate der ein-, zwei- oder dreiwertigen Metallionen der 1. Übergangsmetallreihe des Periodischen Systems und zwar der Gruppen Ib, IIb und VIII z.B. Eisen, Kobalt, Nickel, Kupfer oder Zink, vorzugsweise CuCl, $CuCl_2$, $CoF_2$, $CoCl_2$ und $ZnCl_2$ verwendet.

Bevorzugte Beispiele für Diamine (11) sind tertiäre, tetraalkylsubstituierte aliphatische Diaminverbindungen mit bis zu 4 C-Atomen in der Alkylengruppe (R) und bis zu 6 C-Atomen in der Alkylgruppe ($R^1$ bis $R^4$) sowie heterocyclische Diamine, wie N,N,N′,N′-Tetramethyl- und -ethylmethylendiamin, N,N,N′,N′-Tetramethyl-, -ethyl- und propylethylendiamin, N,N,N′,N′-Tetramethylpropylen- und -isopropylendiamin, N,N,N′,N′-Tetramethylhexylidendiamin sowie Bis(3-methylpiperidino)-methan und N,N′-Dimethylpiperazin. Diese tertiären Diamine (II) sind kommerziell erhältlich oder gemäß J. Am. Chem. Soc. 73, 3518 (1957) bzw. J. Org. Chem. 52, 467 (1987) leicht herstellbar.

Als Verdünnungsmittel dienen aprotische polare Lösemittel wie aliphatische und aromatische Nitrile mit 2 bis 8 C-Atomen, aliphatische Dinitrile mit 5 bis 8 C-Atomen und Polyglykoldialkylether der Formel $R'-(O-CH_2-CHR'')_xOR'$, in der R′ eine Alkylgruppe mit 1 bis 4 C-Atomen, R″ = Wasserstoff oder Methyl und x eine ganze Zahl von 1 bis 6 bedeuten, sowie cyclische Ether. Bevorzugt werden Nitrile oder Dinitrile wie Acetonitril, Propionitril, Butyronitril, Benzonitril oder Adipodinitril sowie Ether wie Tetrahydrofuran, Dioxan, Ethylen- und Propylenglykoldialkylether, insbesondere Ethylengkyoldimethylether und dessen höhere Oligomere oder Mischungen derselben.

Das Metallsalz und das Diamin (11) werden im allgemeinen im äquimolaren Molverhältnis eingesetzt. Die Selektivität für das Säurefluorid (1) mit n= 1 ist unter diesen Bedingungen am größten. Allerdings beeinflussen Unter- bzw. Überschüsse an Diamin (II) im Vergleich zum Metallsalz mit einer Stöchiometrie von 1:2 bis 2:1 den gewünschten Selektivitätsschwerpunkt auf dem dimeren HFPO nur unwesentlich.

Die Konzentration des Metallsalz/Diamin-Komplexes in dem Verdünnungsmittel wird im allgemeinen im Bereich von 0,2-1,2 Mol Metallsalz und 0,2-1,2 Mol Diamin pro Liter Lösemittel eingestellt. Bei Konzentrationen unter 0,1 Mol Metallsalz pro Liter Lösemittel fällt die Aktivität des Katalysators stark ab.

Die Dimerisierung von HFPO mit den erfindungsgemäßen Katalysatoren ist im Temperaturbereich von 0 bis 50°C, vorzugsweise von 5 bis 35°C durchführbar.

Das Katalysatorsystem Metallsalz/Diamin (11) ist schon bei Normaldruck aktiv; ein erhöhter Druck im Reaktionsgefäß wird allerdings im Sinne eines schnelleren Umsatzes bevorzugt. Der Druck im Reaktionsgefäß kann über die Ausströmgeschwindigkeit des gasförmigen oder flüssigen HFPO oder deren Mischungen beeinflußt werden. Insbesondere werden Drucke zwischen 0,5 und 35 bar angewendet.

Das Dimere des HFPO kann bei der Herstellung von Perfluorpropylvinylether Verwendung finden.

In den Beispielen bedeutet % stets Gew.-%.

**Beispiele**

1) In einem Glasautoklaven, der mit Rührer, Thermometer, Monometer und Gaseinleitungsrohr mit Fritte versehen ist, wurden unter Schutzgas 80 g CuCl und 96 g N,N,N′,N′-Tetramethylethylendiamin nacheinander zu 800 ml Acetonitril zugegeben, wobei die zunächst dunkelbraune Lösung eine blaugrüne Farbe annimmt. Nach Schließen des Reaktors und Spülen des Gasraumes mit HFPO wurde unter heftigem Rühren bei 25-30°C HFPO gasförmig in die zuvor bereitete Katalysatorlösung eingeleitet, wobei sich ein Druck von 2,2-2,3 bar einstellt. Nach 105 Minuten wurde die HFPO-Zufuhr unterbrochen, der Rührer abgestellt und dem Reaktionsgemisch Zeit gegeben, sich in zwei Phasen zu trennen. Die untere Produktphase (1178 g) wurde abgetrennt und in Form der Methylester gaschromatographisch analysiert. Sie enthielt 82,9 % 2-Perfluorpropoxy-n-propionylfluorid ((s. Formel (I) mit n= 1), daneben 10,2 % Perfluorpropionylfuorid ((I) mit n= 0) und 6,7 % des Säurefluorids (1) mit n= 2.

2) In einem Reaktionsgefäß, ausgestattet das mit einem Rührer, einem Innenthermometer, einem Monometer, einer Einleitungsfritte und einem Bodenauslaßventil, wurden 60 g CuCl und 72 g N,N,N′,N′-Tetramethylethylendiamin in 1200 ml Acetonitril zu der blaugrünen Katalysatorlösung umgesetzt. Nach Schließen des Reaktionsgefäßes und Spülen des Gasraumes wurde HFPO gasförmig durch die Einleitungsfritte in die Katalysatorlösung mit einer solchen Anströmgeschwindigkeit eingeleitet, daß sich ein Druck von 0,4-0,6 bar einstellt. Die Reaktionstemperatur wurde durch Außenkühlung bei 15-19°C gehalten. Nach 210 Minuten wurde der HFPO-Gasstrom abgestellt und das Rohprodukt (1118 g) wie in Beispiel 1 beschrieben isoliert. Das Gaschromatogramm der Methylester zeigte folgende Zusammensetzung: 81,9 % der Verbindung (I) mit n= 1, 9,6 % (I) mit n= 0 und 8,5 % (I) mit n= 2.

Die im Reaktionsgefäß verbliebene Katalysatorphase wurde anschließend erneut mit gasförmigem HFPO beaufschlagt, dabei wurde die HFPO-Zufuhr stets unterbrochen, wenn das Reaktionsgefäß einen Füllgrad von 90 % erreichte. Nach Trennung von Produkt- und Katalysatorphase und Ablassen der Produktphase durch das Bodenauslaßventil in einen Sammelbehälter wurde die Einleitung von HFPO fortgesetzt. So wurden mit einer HFPO-Anströmgeschwindigkeit von 0,42 kg/Stunde 31,3 kg HFPO in 74 Stunden bei 14-27°C in die Katalysatorlösung geleitet. Das Produkt wurde in 3 Teilmengen gesammelt und analysiert.

| | 1. Reaktionsphase (bis 21 Stunden) | 2. Reaktionsphase (21-44 Stunden) | 3. Reaktionsphase (44-72 Stunden) |
|---|---|---|---|
| (I) n= 0 | 12,0 % | 11,0 % | 12,4 % |
| (I) n= 1 | 80,3 % | 81,2 % | 80,1 % |
| (I) n= 2 | 7,4 % | 7,2 % | 7,4 % |

3) bis 6) Es wurde nach dem in Beispiel 1 angegebenen Verfahren gearbeitet. Tabelle 1 gibt die Bedingungen und die erzielten Ergebnisse an.

7) bis 9) In Tabelle 2 sind die Ansatzmengen der Beispiele, die nach Beispiel 1 durchgeführt worden sind, sowie die erhaltenen Ergebnisse zusammengestellt Als Metallsalze wurden Metallhalogenide mit verschiedenen Kationen eingesetzt.

10) bis 13) Gemäß Beispiel 1 wurden Kupfersalze verschiedener Säuren als Metallsalze eingesetzt. Die Reaktionsbedingungen und Ergebnisse sind in Tabelle 3 zusammengefaßt.

**Tabelle 1:**

| Bsp. | Metallsalz Mol | Diamin Mol | Lösemittel ml | Temperatur °C | Druck bar | Reaktionszeit Minuten | Rohprodukt g | GC-Zusammensetzung (%): (I) n=1 | (I) n=0 | (I) n=2 |
|---|---|---|---|---|---|---|---|---|---|---|
| 3 | CuCl 0,24 | TMMD 0,245 | $CH_3CN$ 500 | 25,30 | 1,4 | 150 | 505 | 75,6 | 11,5 | 12,9 |
| 4 | CuCl 0,4 | TMED 0,41 | $CH_3CN$ 800 | 30 | 0,9 | 105 | 893 | 77,3 | 9,7 | 13,0 |
| 5 | CuCl 0,15 | TMHD 0,15 | $CH_3CN$ 300 | 20 | 1,8-2,0 | 390 | 1137 | 76,6 | 7,7 | 15,7 |
| 6 | CuCl 0,4 | TEED 0,41 | $CH_3CN$ 800 | 32 | 1,0 | 120 | 960 | 80,5 | 8,1 | 11,4 |

TMMD= N,N,N',N'-Tetramethylmethylendiamin
TMED= N,N,N',N'-Tetramethylethylendiamin
TMHD= N,N,N',N'-Tetramethylhexylidendiamin
TEED= N,N,N',N'-Tetraethylethylendiamin

**Tabelle 2:**

| Bsp. | Metallsalz | TMED | Lösemittel | Temperatur | Druck | Reaktionszeit | Rohprodukt | GC-Zusammensetzung (%): (I) n=1 | (I) n=0 | (I) n=2 |
|---|---|---|---|---|---|---|---|---|---|---|
| | Mol | Mol | ml | °C | bar | Minuten | g | | | |
| 7 | CuCl 0,4 | 0,41 | $CH_3CN$ 800 | 12-20 | 0,4 | 330 | 1465 | 81,8 | 5,0 | 12,1 |
| 8 | $CoF_2$ 0,40 | 0,41 | $CH_3CN$ 800 | 27 | 0,4 | 210 | 935 | 78,5 | 6,4 | 15,1 |
| 9 | $ZnCl_2$ 0,38 | 0,41 | $CH_3CN$ 800 | 15 | 1,0 | 180 | 836 | 75,5 | 15,5 | 9,0 |

TMED= N,N,N',N'-Tetramethylethylendiamin

**Tabelle 3:**

| Bsp. | Metallsalz Mol | TMED Mol | Lösemittel ml | Temperatur °C | Druck bar | Reaktionszeit Minuten | Rohprodukt g | GC-Zusammensetzung (%): (I) n=1 | (I) n=0 | (I) n=2 |
|---|---|---|---|---|---|---|---|---|---|---|
| 10 | CuCl 0,4 | 0,41 | $CH_3CN$ 800 | 15 | 0,3 | 135 | 928 | 81 | 6,3 | 12,7 |
| 11 | CuCN 0,57 | 0,60 | $CH_3CN$ 600 | 26-28 | 1,5-1,9 | n.b. | 627 | 80 | 4,0 | 14,0 |
| 12 | CuSCN 0,62 | 0,60 | $CH_3CN$ 600 | 15-19 | 0,8-1,8 | n.b. | 623 | 78 | 11,0 | 12,0 |
| 13 | CuAc 0,2 | 0,2 | $CH_3CN$ 200 | 20 | 1,9 | n.b. | 426 | 65 | 7,0 | 24,0 |

TMED = N,N,N',N'-Tetramethylethylendiamin
AC = Acetat
n.b. = nicht bestimmt

## Patentansprüche

1. Verfahren zur Dimerisierung von hexafluorpropenoxid dadurch gekennzeichnet, daß die Oligomerisation von Hexafluorpropenoxid zu den perfluorierten Carbonsäurefluoriden der allgemeinen Formel

$$C_2F_5(CF_2\text{-}O\text{-}\overset{\displaystyle CF_3}{\overset{|}{C}}F)_nCOF \qquad (I)$$

in der n im wesentlichen die Zahl 1 darstellt, in einem aprotischen polaren Lösungsmittel in Gegenwart eines Kataysators durchgeführt wird, der aus einem Metallsalz der Elemente der 1. Übergangsmetallreihe des Periodischen Systems und einem tertiären Diamin der allgemeinen Formel

$$R^1R^2N\text{-}R\text{-}NR^3R^4 \qquad (II)$$

besteht, in der
R einen unverzweigten oder verzweigten, gesättigten oder ungesättigten Kohlenwasserstoffrest mit 1 bis 12 C-Atomen darstellt, der gegebenenfalls mindestens einen bezüglich der Reaktionskomponenten inerten Heterosubstituenten oder ein Heterokettenglied enthält,
$R^1$ bis $R^4$ unabhängig voneinander cyclische oder acyclische gesättigte oder ungesättigte Kohlenwasserstoffreste mit 1 bis 12C-Atomen sind, die gegebenenfalls jeweils mindestens einen bezüglich der Reaktionskomponenten inerten Heterosubstituenten oder ein Heterokettenglied enthalten, wobei gegebenenfalls jeweils zwei der Reste $R^1$ bis $R^4$ zusätzlich über einen Kohlenwasserstoffrest oder mindestens ein Heteroatom verknüpft sind.

2. Verfahren nach Anspruch 1, dadurch gekennzeichnet, daß als Metallsalze Fluoride, Chloride, Bromide, Jodide sowie Cyanide, Rhodanide oder Acetate des Eisens, Kobalts, Nickels, Kupfers oder Zinks, insbesondere CuCl, $CuCl_2$, $CoF_2$, $CoCl_2$ oder $ZnCl_2$ eingesetzt werden.

3. Verfahren nach Anspruch 1 oder 2, dadurch gekennzeichnet, daß als aprotisches polares Lösungsmittel ein aliphatisches Nitril mit 2 bis 8 Kohlenstoffatomen, ein aliphatisches Dinitril mit 5 bis 8 Kohlenstoffatomen, ein Ether der Formel

$$R'\text{-}(O\text{-}CH_2CHR'')_xOR'$$

in der R′ eine Alkylgruppe mit 1 bis 4 C-Atomen, R″ = Wasserstoff oder Methyl und x eine ganze Zahl von 1 bis 6 bedeuten, oder ein cyclischer Ether, vorzugsweise aber Acetonitril, verwendet wird.

4. Verfahren nach einem oder mehreren der Ansprüche 1 bis 3, dadurch gekennzeichnet, daß das Metallsalz und das tertiäre Diamin (II) im molaren Verhältnis von 2:1 bis 1:2, insbesondere von 1:1 eingesetzt wird.

5. Verfahren nach einem oder mehreren der Ansprüche 1 bis 4, dadurch gekennzeichnet, daß die Oligomerisation bei Temperaturen zwischen 0 und 50°C, vorzugsweise zwischen 5 und 35°C durchgeführt wird.

6. Verfahren nach einem oder mehreren der Ansprüche 1 bis 5, dadurch gekennzeichnet, daß die Konzentration des Metallsalz/Diamin-Komplexes im Lösungsmittel je 0,2 bis 1,2 Mol Metallsalz und Diamin pro Liter Lösungsmittel beträgt.

7. Verfahren nach einem oder mehreren der Ansprüche 1 bis 6, dadurch gekennzeichnet, daß die Umsetzung bei Normaldruck oder erhöhtem Druck bei Werten zwischen 0,5 und 3,5 bar erfolgt.

8. Verfahren nach einem oder mehreren der Ansprüche 1 bis 7, dadurch gekennzeichnet, daß im Diamin (II) der Rest R 1 bis 4 C-Atome und die Reste $R^1$ bis $R^4$ 1 bis 6 C-Atome aufweisen.

9. Verfahren nach einem oder mehreren der Ansprüche 1 bis 8, dadurch gekennzeichnet, daß als Heteroatom, Heterosubstituent oder Heterokettenglied Sauerstoff oder Stickstoff vorhanden ist.

## Claims

1. A process for the dimerization of hexafluoropropene oxide, which comprises the oligomerization of hexafluoropropene oxide to give the perfluorinated carbonyl fluorides of the general formula

$$C_2F_5(CF_2\text{-}O\text{-}\underset{\displaystyle |}{\overset{CF_3}{CF}})_nCOF \qquad (I)$$

in which n substantially represents the number 1, being carried out in an aprotic polar solvent in the presence of a catalyst which is composed of a metal salt of the elements of the Ist transition metal series of the periodic table and of a tertiary diamine of the general formula

$$R^1R^2N\text{-}R\text{-}NR^3R^4 \qquad (II)$$

in which R represents an unbranched or branched, saturated or unsaturated hydrocarbon radical which has 1 to 12 carbon atoms and optionally contains at least one hetero chain member or at least one hetero substituent which is inert toward the reaction components, $R^1$ to $R^4$ are, independently of one another, cyclic or acyclic saturated or unsaturated hydrocarbon radicals which have 1 to 12 carbon atoms and optionally each contain at least one hetero chain member or at least one hetero substituent which is inert toward the reaction components, with, in addition, each two of the radicals $R^1$ to $R^4$ optionally being linked via a hydrocarbon radical or at least one hetero atom.

2. The process as claimed in claim 1, wherein fluorides, chlorides, bromides, iodides and cyanides, thiocyanates or acetates of iron, cobalt, nickel, copper or zinc, in particular CuCl, $CuCl_2$, $CoF_2$, $CoCl_2$ or $ZnCl_2$, are employed as metal salts.

3. The process as claimed in claim 1 or 2, wherein an aliphatic nitrile having 2 to 8 carbon atoms, an aliphatic dinitrile having 5 to 8 carbon atoms, an ether of the formula

$$R'\text{-}(O\text{-}CH_2CHR'')_xOR'$$

in which R′ denotes an alkyl group having 1 to 4 carbon atoms, R″ denotes hydrogen or methyl and x denotes an integer from 1 to 6, or a cyclic ether, but preferably acetonitrile, is used as aprotic polar solvent.

4. The process as claimed in one or more of claims 1 to 3, wherein the metal salt and the tertiary diamine (II) are employed in the molar ratio of 2:1 to 1:2, in particular of 1:1.

5. The process as claimed in one or more of claims 1 to 4, wherein the oligomerization is carried out at temperatures between 0 and 50°C, preferably between 5 and 35°C.

6. The process as claimed in one or more of claims 1 to 5, wherein the concentration of the metal salt/diamine complex in the solvent is 0.2 to 1.2 mole of metal salt and of diamine per liter of solvent.

7. The process as claimed in one or more of claims 1 to 6, wherein the reaction is carried out under atmospheric pressure or elevated pressure of between 0.5 and 3.5 bar.

8. The process as claimed in one or more of claims 1 to 7, wherein in the diamine (II) the radical R has 1 to 4 carbon atoms and the radicals $R^1$ to $R^4$ have 1 to 6 carbon atoms.

9. The process as claimed in one or more of claims 1 to 8, wherein oxygen or nitrogen is present as hetero atom, hetero substituent or hetero chain member.

## Revendications

1. Procédé pour dimériser l'oxyde d'hexafluoropropène, caractérisé en ce qu'on effectue l'oligomérisation de l'oxyde d'hexafluoropropène conduisant aux fluorures d'acides carboxyliques perfluorés répondant à la formule générale I :

$$C_2F_5(CF_2\text{-}O\text{-}\underset{}{\overset{CF_3}{\overset{|}{C}}}F)_nCOF \qquad (I)$$

dans laquelle n représente essentiellement le nombre 1, dans un solvant polaire aprotique, en présence d'un catalyseur constitué d'un sel métallique d'un des éléments de la première série des métaux de transition de la classification périodique et d'une diamine tertiaire répondant à la formule générale II :

$$R^1R^2N\text{-}R\text{-}NR^3R^4 \qquad (II)$$

dans laquelle

R représente un radical hydrocarboné saturé ou insaturé, ramifié ou non ramifié, contenant de 1 à 12 atomes de carbone, qui contient éventuellement un ou plusieurs substituants à hétéro-atome, inertes à l'égard des composantes réactionnelles, ou un chaînon à hétéro-atome, et

$R^1$ à $R^4$ représentent chacun, indépendamment l'un de l'autre, un radical hydrocarboné saturé ou insaturé, cyclique ou acyclique, contenant de 1 à 12 atomes de carbone, qui contient éventuellement un ou plusieurs substituants à hétéro-atome inertes à l'égard des composantes réactionnelles ou un chaînon à hétéroatome,

deux des symboles $R^1$ à $R^4$ pouvant à chaque fois être en outre reliés par un radical hydrocarboné ou par au moins un hétéro-atome.

**2.** Procédé selon la revendication 1 caractérisé en ce qu'on utilise, comme sels métalliques, des fluorures, des chlorures, des bromures, des iodures, des cyanures, des thiocyanates ou des acétates du fer, du cobalt, du nickel, du cuivre ou du zinc, plus particulièrement CuCl, $CuCl_2$, $CoF_2$, $CoCl_2$ ou $ZnCl_2$.

**3.** Procédé selon l'une des revendications 1 et 2 caractérisé en ce qu'on utiliser comme solvant polaire aprotique, un nitrile aliphatique contenant de 2 à 8 atomes de carbone, un dinitrile aliphatique contenant de 5 à 8 atomes de carbone, un éther répondant à la formule :

$$R'\text{-}(O\text{-}CH_2\text{-}CHR'')_x\text{-}OR'$$

dans laquelle R′ représente un alkyle contenant de 1 à 4 atomes de carbone, R" l'hydrogène ou un méthyle et x un nombre entier de 1 à 6, ou un éther cyclique, mais de préférence l'acétonitrile.

**4.** Procédé selon l'une des revendications 1 à 3, caractérisé en ce que le sel métallique et la diamine tertiaire (II) sont mis en jeu dans un rapport molaire compris entre 2:1 et 1:2, plus particulièrement égal à 1:1.

**5.** Procédé selon une ou plusieurs des revendications 1 à 4, caractérisé en ce que l'oligomérisation est effectuée à des températures comprises entre 0 et 50 °C, de préférence entre 5 et 35 °C.

**6.** Procédé selon une ou plusieurs des revendications 1 à 5, caractérisé en ce que la concentration en le complexe sel métallique/diamine dans le solvant est comprise, pour chacun de ces deux constituants, entre 0,2 et 1,2 mol par litre du solvant.

**7.** Procédé selon l'une quelconque des revendications 1 à 6, caractérisé en ce que la réaction est effectuée sous la pression normale ou sous pression élevée, à des valeurs comprises entre 0,5 et 3,5 bar.

**8.** Procédé selon l'une quelconque des revendications 1 à 7, caractérisé en ce que, dans la diamine (II), le radical R contient de 1 à 4 atomes de carbone et les radicaux $R^1$ à $R^4$ contiennent chacun de 1 à 6 atomes de carbone.

**9.** Procédé selon l'une quelconque des revendications 1 à 8, caractérisé en ce que l'hétéro-atome, l'hétéroatome du substituant ou l'hétéro-atome du chaînon à hétéro-atome est l'oxygène ou l'azote.